# EUROPEAN PATENT SPECIFICATION

(11) **EP 1 655 051 B1**
(45) Date of publication and mention of the grant of the patent: **09.05.2012**
(21) Application number: 05253932.7
(22) Date of filing: 24.06.2005
(51) Int. Cl.: A61M 21/00, A61B 7/00, A61F 5/56, A61M 16/00, A61B 5/00

(54) **Rising-alarm generating apparatus and method**
Gerät und Verfahren zur Alarmauslösung
Appareil et méthode pour déclencher une alarme

(30) Priority: 08.11.2004 KR 2004090350
(43) Date of publication of application: 10.05.2006
(73) Proprietor: Samsung Electronics Co., Ltd., Suwon-si, Gyeonggi-do (KR)
(72) Inventor: Jang, Woo-young, Seoul (KR); Kim, Yeon-bae, Gyeonggi-do (KR); Park, Jae-chan, Gyeonggi-do (KR); Park, Jeong-je, Gyeonggi-do (KR)
(74) Representative: Anderson, James Edward George

(56) References cited:
- EP-A- 0 493 719
- WO-A-02/13677
- WO-A-87/05494
- WO-A-2004/006764
- DE-A1- 19 811 206
- DE-A1- 19 904 260
- GB-A- 2 103 807
- US-A1- 2004 127 198

## Description

The present invention relates to an apparatus for monitoring the sleep of a person, and more particularly to a rising-alarm generating apparatus and method, which can wake a person by detecting their snore sounds.

Snoring occurs when a sleeper's throat narrows and prevents air from passing in and out easily. Snoring indicates respiratory distress while sleeping.

25 to 45% of normal adults snore. When the airway is completely blocked by severe muscle relaxation during sleep, heavy obesity, or other causes, air cannot reach the lungs. This phenomenon is called sleep apnea, and appears in 5 to 10% of normal adults. Sleep apnea prevents the lungs from obtaining fresh air, and when the brain senses such a state, it wakes the body and contracts muscles to open the airway, allowing the lungs to breathe again.

Since snorers suffer from lack of sleep, they are not refreshed in the morning and easily get sleepy during the day. Therefore, they can have poor concentration and achievement. In addition, they are at risk of car accidents. Socially, snorers easily become passive and introspective in human relations. When severe snoring develops into sleep apnea, sufferers can be easily attacked by heart diseases such as hypertension, angina pectoris, myocardial infarction, etc. or adult diseases such as diabetes, liver failure, etc.

In the past, medical treatments and correction of sleeping postures were used to suppress snoring and sleep apnea.

However, the sleeping posture is hard to control while asleep, and snoring or sleep apnea cannot be treated without the help of others.

Even those unaffected by snoring or sleep apnea can fall asleep during study or work, and it is difficult for them to get up at the intended time without the help of others.

DE 19904260 A1 discloses spectacles having sensors, including a microphone, for detecting symptoms of sleep disturbance. A processor analyses an output of the sensors and sends a trigger signal to a waking light element in the spectacles when sleep disturbance is detected.

The invention provides a rising-alarm generating apparatus according to claim 1 and a rising alarm generating method according to claim 18.

The present invention thus provides a rising-alarm generating apparatus which can sense a user's sleeping state and wake the user.

The present invention also provides a rising-alarm generating method which can sense a user's sleeping state and wake the user.

The above and other features and advantages of the present invention will become more apparent by describing in detail exemplary embodiments thereof with reference to the attached drawings in which:
FIG. 1 is a block diagram illustrating a rising-alarm generating apparatus according to an embodiment of the present invention;
FIGS. 2A to 2C are diagrams illustrating how an alarm-signal output unit of a rising-alarm generating apparatus provided in a mobile phone can be separated from the mobile phone according to an embodiment of the present invention; and
FIG. 3 is a flowchart illustrating a rising-alarm generating method according to an embodiment of the present invention.

Hereinafter, a rising-alarm generating apparatus according to the present invention will be described in detail with reference to the accompanying drawings

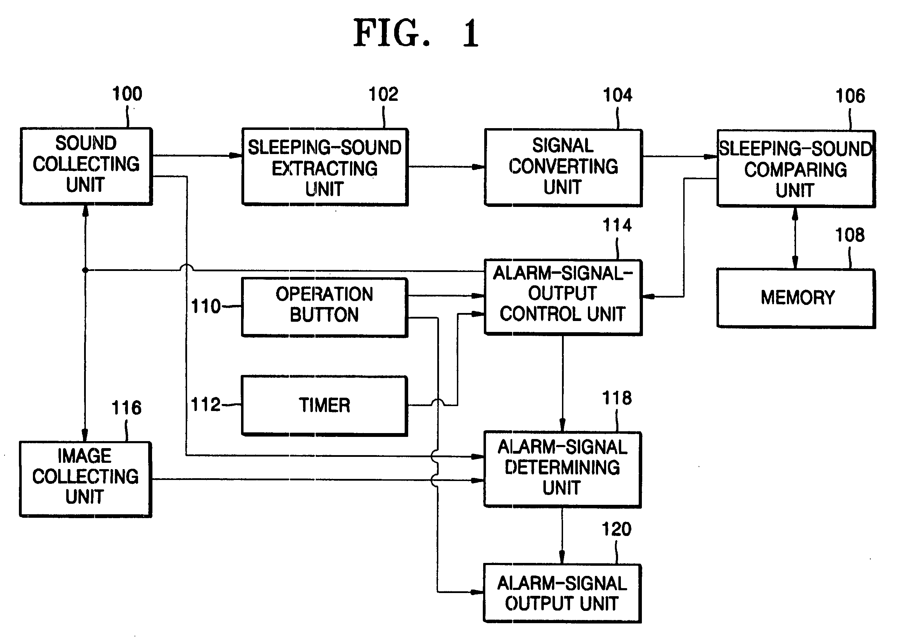

FIG. 1 is a block diagram illustrating a rising-alarm generating apparatus according to an embodiment of the present invention. The rising-alarm generating apparatus includes a sound collecting unit 100, a sleeping-sound extracting unit 102, a signal converting unit 104, a sleeping-sound comparing unit 106, a memory 108, an operation button 110, a timer 112, an alarm-signal-output control unit 114, an image collecting unit 116, an alarm-signal determining unit 118, and an alarm-signal output unit 120.

The sound collecting unit 100 collects peripheral sounds and outputs the collected sounds to the sleeping-sound extracting unit 102. The sound collecting unit 100 may be a device such as a microphone which can collect peripheral human voices, sounds, noises, etc. The sound collecting unit 100 performs a sound collecting function in response to a control signal indicating operation start from the alarm-signal-output control unit 114 to be described in detail later.

The sleeping-sound extracting unit 102 extracts a human sleeping sound from the collected sounds, in response to the input of the sounds collected by the sound collecting unit 100, and then outputs the extracted sleeping sound to the signal converting unit 104. The sleeping sound includes sounds of constant patterns, which can be generated during a person's sleep, such as the sounds of snoring and sleep apnea. The sound of snoring is emitted when a sleeper's throat narrows and prevents air from passing in and out easily. The sound of sleep apnea is emitted when the airway is completely blocked and air cannot reach the lungs. That is, the sound of sleep apnea is a respiratory pattern generated when the brain senses the throat biockage and wakes the body to recover breathing.

The sleeping-sound extracting unit 102 extracts the sleeping sound by using an analog filter to remove sounds other than the sleeping sounds such as the sound of snoring or sleep apnea from the collected sounds.

The sleeping-sound extracting unit 102 preferably amplifies the extracted sleeping sound. That is, the sleeping-sound extracting unit 102 amplifies the extracted sleeping sound using an amplifier and then outputs the amplified sleeping sound to the signal converting unit 104.

The signal converting unit 104 converts the sleeping sound input from the sleeping-sound extracting unit 102 into a digital signal. The signal converting unit 104 converts the analog sleeping sound signal into a digital signal for a digital process and then outputs the converted sleeping sound to the sleeping-sound comparing unit 106.

The sleeping-sound comparing unit 106 compares the converted sleeping sound with a user's sleeping sound sample stored in advance, to check the similarity. The sleeping sound sample is the sound of the user's snoring or sleep apnea which is stored in advance in a memory space by the user of the rising-alarm generating apparatus. The user can intentionally make and store his sound of snoring or sleep apnea in advance, on the assumption that he is in a sleeping state. The user's sleeping sound sample is in a digital form. If the sleeping sound converted into a digital signal is similar to the user's sleeping sound sample, it means that the person making the sleeping sound is the user of the rising-alarm generating apparatus.

The sleeping-sound comparing unit 106 may store and manage the user's sleeping sound sample in an internal memory of the sleeping-sound comparing unit 106, or in a particular memory 108 as shown in FIG. 1. When receiving the sleeping sound converted into a digital signal from the signal converting unit 104, the sleeping-sound comparing unit 106 reads out the user's sleeping sound sample from the memory 108 in response to the sleeping sound, compares the sleeping sound input from the signal converting unit 104 with the sleeping sound read out from the memory 108 to check the similarity, and then outputs the comparison result to the alarm-signal-output control unit 114.

The memory 108 is a storage space for storing the user's sleeping sound sample. Such a memory 108 may be embodied as a particular memory or may be provided in the sleeping-sound comparing unit 106.

The operation button 110 allows the user to input an operation starting instruction and an operation ending instruction to the rising-alarm generating apparatus. The user gives the operation starting instruction or the operation ending instruction of the rising-alarm generating apparatus according to the present invention through the operation button 110. Since the power consumption is higher when the rising-alarm generating apparatus is always turned on, the user can determine the operation of the rising-alarm generating apparatus by using the operation button 110. When the operation starting instruction or the operation ending instruction is input through the operation button 110 by the user, the operation starting instruction or the operation ending instruction is output to the alarm-signal-output control unit 114.

On the other hand, the user may give the rising-alarm generating apparatus an output stopping instruction indicating that the output of the rising-alarm signal from the alarm-signal output unit 120 should be stopped, through the operation button 110. That is, when the rising-alarm signal is being output, the awoken user should be able to stop the output of the rising-alarm signal. The output stopping instruction for stopping the output of the rising-alarm signal is input through the operation button 110. When the output stopping instruction is input through the operation button 110 from the user, the output stopping instruction is output to the alarm-signal output unit 120.

The timer 112 supplies time information to the alarm-signal-output control unit 114, such that the alarm-signal-output control unit 114 can control the operation of the rising-alarm generating apparatus over time.

The alarm-signal-output control unit 114 can control operation start and operation end of the rising-alarm generating apparatus, in response to the operation starting instruction and the operation ending instruction. The user can input the operation start instruction and the operation end instruction to the rising-alarm generating apparatus through the operation button 110. The operation start instruction and the operation end instruction from the user are transmitted to the alarm-signal-output control unit 114. The alarm-signal-output control unit 114 controls the operation start and the operation end of the rising-alarm generating apparatus in response to the transmitted operation starting instruction and operation ending instruction.

The alarm-signal-output control unit 114 may control the operation start and the operation end of the rising-alarm generating apparatus for a predetermined time period at predetermined intervals, by using the timer 112. For example, the alarm-signal-output control unit 114 may be able to allow the rising-alarm generating apparatus to operate for 5 minutes at intervals of 20 minutes by using the time information supplied from the timer 112, and not operate in the 15 minute periods outside the times described above. Further, for example, the alarm-signal-output control unit 114 may be able to allow the rising-alarm generating apparatus to operate for 6 hours from midnight to 6 O'clock at intervals of 24 hours by using the time information supplied from the timer 112, and not to operate for the 18 hour periods outside the times described above.

At this time, the alarm-signal-output control unit 114 preferably controls the operation start and the operation end of the sound collecting unit 100 and the image collecting unit 116 collecting peripheral images correspondingly to the operation start and the operation end of the rising-alarm generating apparatus. That is, when the rising-alarm generating apparatus starts its operation under the alarm-signal-output control unit 114, the sound collecting unit 100 and the image collecting unit 116 receive a control signal for operation start from the alarm-signal-output control unit 114.

The sound collecting unit 100 collects peripheral sounds after receiving the control signal for the operation start. The peripheral sounds collected by the sound collecting unit 100 are output to the alarm-signal determining unit 118.

The image collecting unit 116 collects peripheral images after receiving the control signal for operation start. The peripheral images collected by the image collecting unit 100 are output to the alarm-signal determining unit 118. For example, the image collecting unit 116 may be a camera. At this time, since a large amount of image information is collected by the image collecting unit 116, the image collecting unit 116 preferably collects only a predetermined number of image cuts for a predetermined time period.

The sound collecting unit 100 and the image collecting unit 116 stop their operations after receiving the control signal for operation end.

The alarm-signal-output control unit 114 outputs a control signal for controlling the output of the alarm-signal output unit 120, to the alarm-signal determining unit 118, in accordance with the comparison result of the sleeping-sound comparing unit 106. When the sleeping-sound comparing unit 106 indicates that the sleeping sound converted into a digital signal is similar to the user's sleeping sound sample, the alarm-signal-output control unit 114 determines that the person emitting the sleeping sound is the user of the rising-alarm generating apparatus, and outputs to the alarm-signal determining unit 118 a control signal indicating that the person emitting the sleeping sound should be woken.

In addition, the alarm-signal-output control unit 114 preferably controls the alarm-signal output unit 120 to continuously output the rising-alarm signal while the sleeping sound is continuously collected. The continuous collection of the sleeping sound means that the user stays asleep. Therefore, the alarm-signal-output control unit 114 outputs the control signal such that the rising-alarm signal can be continuously output until the user wakes up.

The alarm-signal determining unit 118 determines the kind of rising-alarm signal output from the alarm-signal output unit 120 on the basis of the peripheral environment information, in response to the control signal from the alarm-signal-output control unit 114.

When receiving the control signal from the alarm-signal-output control unit 114, the alarm-signal determining unit 118 receives the peripheral sound information collected by the sound collecting unit 100 and the peripheral image information collected by the image collecting unit 116, and detects the peripheral environment information therefrom.

When determining that it is dark on the basis of the detected peripheral environment information, the alarm-signal determining unit 118 determines an optical signal as the rising-alarm signal. The optical signal includes a flashing stimulation.

When determining that it is silent on the basis of the detected peripheral environment information, the alarm-signal determining unit 118 determines a sound signal as the rising-alarm signal. Here, the sound signal is preferably not loud enough to annoy others. A meiody may used as the sound signal.

When determining that it is noisy on the basis of the detected peripheral environment information, the alarm-signal determining unit 118 determines a vibration signal as the rising-alarm signal.

The alarm-signal determining unit 118 can determine one or more of the sound signal, the vibration signal, and the optical signal described above as the rising-alarm signal.

On the other hand, the alarm-signal determining unit 118 may determine the kind of rising-alarm signal in accordance with the user's selection. That is, the rising-alarm signal may be determined in advance in accordance with the user's selection, not the above-mentioned peripheral environment information.

The alarm-signal output unit 120 outputs the rising-alarm signal to wake the user. The alarm-signal output unit 120 outputs one or more of the sound signal, the vibration signal, and the optical signal determined by the alarm-signal determining unit 118 as the rising-alarm signal. The alarm-signal output unit 120 continuously outputs the rising-alarm signal until the sleeping sound is not collected, that is, until the user is awake, in response to the control signal of the alarm-signal-output control unit indicating the continuous output of the rising-alarm signal.

When the user wakes up, the user can give the output stopping instruction allowing the output of the rising-alarm signal to be stopped, through the above-mentioned operation button 110. When receiving the output stopping instruction, the alarm-signal output unit 120 stops the output of the rising-alarm signal in response to the output stopping instruction.

The rising-alarm generating apparatus is preferably provided in a mobile phone, a notebook computer, or a personal digital assistant (PDA).

Here, when the rising-alarm generating apparatus is provided in the mobile phone or the personal digital assistant and radio communication is required during the operation of the rising-alarm generating apparatus, the alarm-signal-output control unit 114 preferably stops the operation of the rising-alarm generating apparatus. For example, it is assumed that the rising-alarm generating apparatus is provided in the mobile phone, and the rising-alarm generating apparatus is operating, when an audio call is received from another party by wireless network. Then, the alarm-signal-output control unit 114 temporarily stops the operation of the rising-alarm generating apparatus and permits the audio communication as an intrinsic function of the mobile phone.

On the other hand, when the rising-alarm generating apparatus is provided in the mobile phone, notebook computer, or personal digital assistant, the alarm-signal output unit 120 can be separated from the rising-alarm generating apparatus. In this case, the alarm-signal output unit 120 outputs the rising-alarm signal through the radio communication with the rising-alarm generating apparatus.

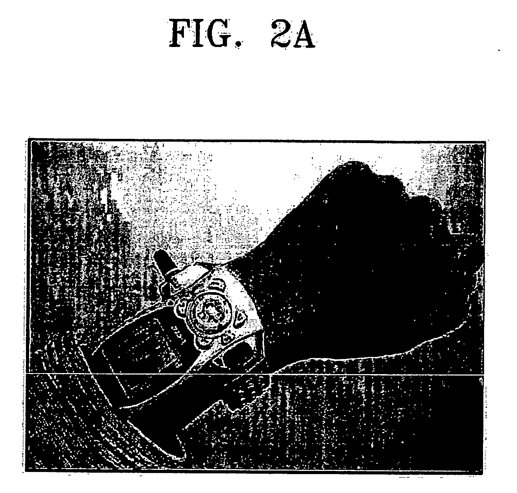

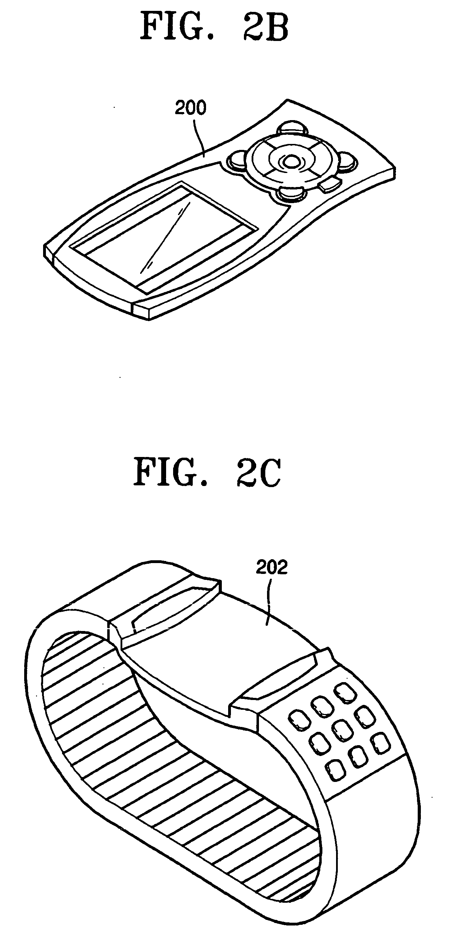

FIGS. 2A to 2C are diagrams illustrating how the alarm-signal output unit 120 of the rising-alarm generating apparatus provided in a mobile phone can be separated from the mobile phone. FIG. 2A is a diagram illustrating an example of a mobile phone 200 and an alarm-signal output unit 202 coupled to the mobile phone 200, mounted on a wrist. FIG. 2B is a diagram illustrating an example of the alarm-signal output unit 202 separated from the mobile phone 200, and FIG. 2C is a diagram illustrating an example of the mobile phone 200 separated from the alarm-signal output unit 202.

When a control signal indicating the output of the rising-alarm signal is transmitted to the alarm-signal output unit 202 put onto the wrist from the alarm-signal-output control unit 114 provided in the mobile phone 200 shown in FIG. 2C by wireless communication, the alarm-signal output unit 202 outputs a rising-alarm signal such as a sound signal, an optical signal, or a vibration signal in accordance with the transmitted control signal.

A rising-alarm generating method according to the present invention will now be described in detail with reference to the accompanying drawings.

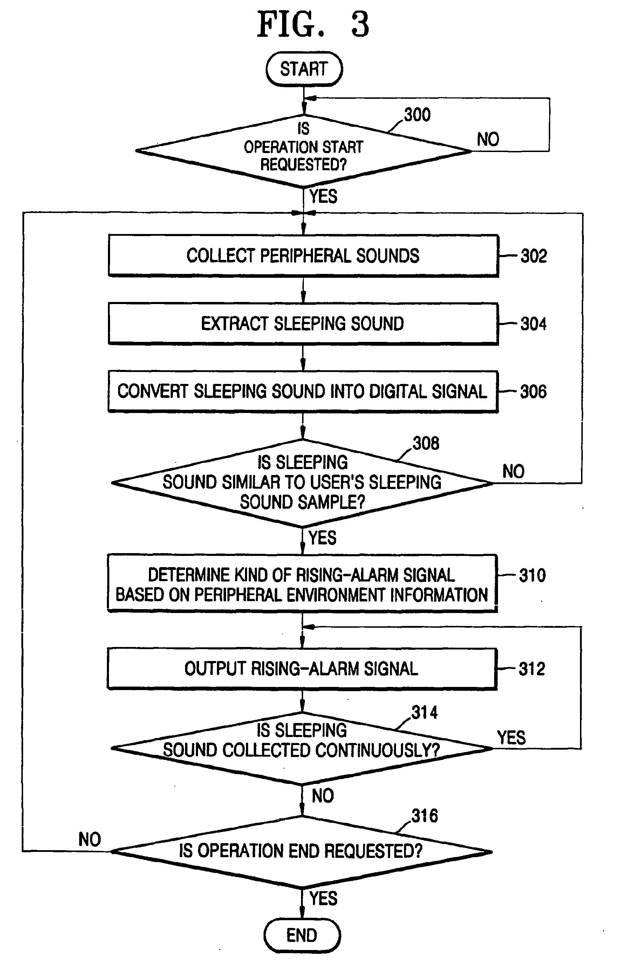

FIG. 3 is a flowchart illustrating a rising-alarm generating method according to an embodiment of the present invention.

First, it is checked whether the operation start of the rising-alarm generating apparatus is requested (operation 300). The operation start or the operation end of the rising-alarm generating apparatus may be automatically performed for a predetermined time period at predetermined intervals, or the operation start or the operation end of the rising-alarm generating apparatus may be performed in response to the operation starting instruction or the operation ending instruction from a user.

If the operation start of the rising-alarm generating apparatus is requested, the peripheral sounds are collected (operation 302).

After operation 302, the human sleeping sound is extracted from the collected sounds (operation 304). The sleeping sound includes sounds of constant pattern, which can be generated when a person sleeps, such as snoring and sleep apnea. The sleeping sound is extracted by removing sounds other than the sleeping sound from the collected sounds. At this time, the extracted sleeping sound is preferably amplified.

After operation 304, the extracted sleeping sound is converted into a digital signal (operation 306).

After operation 306, the converted sleeping sound is compared with a user's sleeping sound sample stored in advance, to check similarity (operation 308). The sleeping sound sample is the sound of the user's snoring or sleep apnea, which is stored in advance in a memory space by the user of the rising-alarm generating apparatus. The user can intentionally make and store his sound of snoring or sleep apnea in advance, on the assumption that he is in a sleeping state. The user's sleeping sound sample is in a digital form.

If the sleeping sound is similar to the user's sleeping sound sample, the kind of rising-alarm signal is determined in accordance with the peripheral environment information (operation 310). If the sleeping sound converted into a digital signal is similar to the user's sleeping sound sample, it means that the person emitting the sleeping sound is the user of the rising-alarm generating apparatus.

The peripheral environment information is detected from the peripheral image information and the peripheral sound information. When it is determined that it is dark on the basis of the detected peripheral environment information, an optical signal is determined as the rising-alarm signal. When it is determined that it is silent on the basis of the detected peripheral environment information, a sound signal is determined as the rising-alarm signal. When it is determined that it is noisy on the basis of the detected peripheral environment information, a vibration signal is determined as the rising-alarm signal.

One or more of the sound signal, the vibration signal, and the optical signal described above can be determined as the rising-alarm signal.

On the other hand, the kind of rising-alarm signal may be determined in accordance with the user's selection. That is, the rising-alarm signal may be determined in advance in accordance with the user's selection, not the above-mentioned peripheral environment information.

After operation 310, the rising-alarm signal is output (operation 312). One or more of the sound signal, the vibration signal, and the optical signal is preferably output as the rising-alarm signal.

After operation 312, it is checked whether the sleeping sound is continuously collected (operation 314). The continuous collection of the sleeping sound means that the user stays asleep. When the sleeping sound is continuously collected, operation 312 is repeatedly performed so as to continuously output the rising-alarm signal.

However, when the sleeping sound is not continuously collected, it is checked whether the operation end of the rising-alarm generating apparatus is requested (operation 316). As described above, the operation start or the operation end of the rising-alarm generating apparatus may be automatically performed for a predetermined time period at predetermined intervals, or the operation start or the operation end of the rising-alarm generating apparatus may be performed in response to the operation starting instruction or the operation ending instruction from the user. Therefore, when the operation end is not requested, the procedure is advanced to operation 302, and the above-mentioned operations are performed again, but when the operation end is requested, the procedure is finished.

The aforementioned rising-alarm generating method is preferably carried out in a mobile phone, a notebook computer, or a personal digital assistant.

Here, when the rising-alarm generating apparatus is provided in the mobile phone or the personal digital assistant, and radio communication of the mobile phone or the personal digital assistant is required while performing the respective operations of the rising-alarm generating method, it is preferable that the corresponding operation is temporarily stopped.

While the present invention has been particularly shown and described with reference to the exemplary embodiments of the rising-alarm generating apparatus and method, these embodiments exemplify the present invention. It will be understood by those skilled in the art that various changes in form and details may be made therein without departing from the scope of the present invention as defined by the following claims.

As described above, the rising-alarm generating apparatus and method according to the present invention can allow a user to get up by sensing the user's undesirable sleeping state.

When a user snores or shows sleep apnea due to incorrect postures, etc., it is possible to correct the user's posture and to suppress the snore or the sleep apnea.

## Claims

1. A rising-alarm generating apparatus comprising:
a sound collecting unit (100) for collecting peripheral sounds;
a sleeping-sound extracting unit (102) for extracting a human sleeping sound from the collected sounds; and
an alarm-signal output unit (120) for outputting a rising-alarm signal for waking the user;
wherein the apparatus is **characterized in that** it further comprises:
a signal converting unit (104) for converting the extracted sleeping sound into a digital signal;
a sleeping-sound comparing unit (106) for comparing the converted sleeping sound with a user's sleeping sound sample stored in advance to check for similarity;
an alarm-signal-output control unit (114) for controlling the output of the alarm-signal output unit (120) in accordance with the comparison result of the sleeping-sound comparing unit (106); and
an alarm-signal determining unit (118) arranged to determine a kind of rising-alarm signal output from the alarm-signal output unit (120) on the basis of peripheral environment information in response to a control signal from the alarm-signal-output control unit (114).

2. The rising-alarm generating apparatus according to claim 1, wherein the sleeping-sound extracting unit (102) is arranged to extract a human sound of snoring or sleep apnea as the sleeping sound.

3. The rising-alarm generating apparatus according to claim 1 or 2, wherein the sleeping-sound extracting unit (102) is arranged to amplify the extracted sleeping sound.

4. The rising-alarm generating apparatus according to any preceding claim, wherein the sleeping-sound comparing unit (106) is arranged to store and manage the user's sleeping-sound sample in an inner memory (108) or in a particular memory.

5. The rising-alarm generating apparatus according to any preceding claim, wherein the alarm-signal output unit (120) is arranged to output a sound signal, a vibration signal, or an optical signal as the rising-alarm signal.

6. The rising-alarm generating apparatus according to any preceding claim, wherein the alarm-signal-output control unit (114) is arranged to control the operation start and operation end of the rising-alarm generating apparatus for a predetermined time period at predetermined intervals.

7. The rising-alarm generating apparatus according to claim 6, wherein the alarm-signal-output control unit (114) is arranged to control the operation start and operation end of the sound collecting unit (100) and the image collecting unit (116), in response to the operation start and the operation end of the rising-alarm generating apparatus.

8. The rising-alarm generating apparatus according to any preceding claim, wherein the alarm-signal-output control unit (114) is arranged to control the alarm-signal output unit (120) to continuously output the rising-alarm signal while the sleeping sound is continuously collected.

9. The rising-alarm generating apparatus according to any preceding claim, further comprising an operation button (110) for inputting an operation starting instruction and an operation ending instruction from the user.

10. The rising-alarm generating apparatus according to claim 9, wherein the alarm-signal-output control unit (114) is arranged to control the operation start and operation end of the rising-alarm generating apparatus in response to the operation starting instruction and the operation ending instruction input through the operation button (110).

11. The rising-alarm generating apparatus according to claim 9, wherein the operation button (110) serves to input an output stopping instruction from the user indicating that the output of the rising-alarm signal from the alarm-signal output unit (120) should be stopped.

12. The rising-alarm generating apparatus according to claim 1, wherein the alarm-signal determining unit is arranged to determine the kind of rising-alarm signal in accordance with the user's selection.

13. The rising-alarm generating apparatus according to claim 1, wherein the alarm-signal determining unit is arranged to detect the peripheral environment information from image information collected by an image collecting unit for collecting peripheral images and sound information collected by the sound collecting unit.

14. The rising-alarm generating apparatus according to claim 13, wherein the alarm-signal determining unit (118) is arranged to determine an optical signal as the rising-alarm signal when it is dark.

15. The rising-alarm generating apparatus according to claim 13 or 14, wherein the alarm-signal determining unit (118) is arranged to determine a sound signal as the rising-alarm signal when it is silent.

16. The rising-alarm generating apparatus according to any of claims 13 to 15, wherein the alarm-signal determining unit (118) is arranged to determine a vibration signal as the rising-alarm signal when it is noisy.

17. A mobile phone, a notebook computer, or a personal digital assistant comprising the rising-alarm generating apparatus according to any preceding claim.

18. The device according to claim 17, wherein the alarm-signal-output control unit (114) is arranged to temporarily stop the operation of the rising-alarm generating apparatus when the device is a mobile phone or a personal digital assistant, and radio communication is requested during the operation of the rising-alarm generating apparatus.

19. The device according to claim 17 or 18, wherein the alarm-signal output unit (120) can be separated from the rising-alarm generating apparatus, and is arranged to output the rising-alarm signal by performing radio communication with the rising-alarm generating apparatus.

20. A rising-alarm generating method comprising:
a. collecting peripheral sounds (302);
b. extracting a human sleeping sound from the collected sounds (304);
c. converting the extracted sleeping sound into a digital signal (306);
d. comparing the converted sleeping sound with a user's sleeping sound sample stored in advance, to check for similarity (308); and
e. outputting a rising-alarm signal for waking the user when the sleeping sound is similar to the user's sleeping sound sample (312),
wherein the method further comprises, between d. and e.:
f. determining a kind of rising-alarm signal on the basis of the peripheral environmental information (310).

21. The rising-alarm generating method according to claim 20, wherein, in b., a human sound of snoring or sleep apnea is extracted as the sleeping sound.

22. The rising-alarm generating method according to claim 20 or 21, wherein, in b., the extracted sleeping sound is amplified.

23. The rising-alarm generating method according to any of claims 20 to 22, wherein, in e., a sound signal, a vibration signal, or an optical signal is output as the rising-alarm signal.

24. The rising-alarm generating method according to claim 23, wherein, in f., the kind of rising-alarm signal is determined in accordance with the user's selection.

25. The rising-alarm generating method according to claim 23, wherein, in f., the peripheral environmental information is detected from image information and sound information of the peripheral environment.

26. The rising-alarm generating method according to claim 25, wherein, in f., an optical signal is determined as the rising-alarm signal when it is dark.

27. The rising-alarm generating method according to claim 25 or 26, wherein, in f., a sound signal is determined as the rising-alarm signal when it is silent.

28. The rising-alarm generating method according to any of claims 25 to 27, wherein, in f., a vibration signal is determined as the rising-alarm signal when it is noisy.

29. The rising-alarm generating method according to any one of claims 20 to 28, further comprising:
checking whether the sleeping sound is continuously collected, to be performed after e.

30. The rising-alarm generating method according to any of claims 20 to 29, wherein the rising-alarm generating method is performed for a predetermined time period at predetermined intervals.

31. The rising-alarm generating method according to any of claims 20 to 30, wherein the operation of the rising-alarm generating method is started and ended in response to an operation starting instruction and an operation ending instruction from the user.

32. The rising-alarm generating method according to any of claims 20 to 31, wherein the rising-alarm generating method is executed by a mobile phone, a notebook computer, or a personal digital assistant.

## Patentansprüche

1. Alarmerzeugungsvorrichtung, die Folgendes umfasst:
eine Geräuschaufnahmeeinheit (100) zum Aufnehmen von peripheren Geräuschen;
eine Schlafgeräuschextraktionseinheit (102) zum Extrahieren eines menschlichen Schlafgeräuschs aus den aufgenommenen Geräuschen; und
eine Alarmsignalausgabeeinheit (120) zum Ausgeben eines Alarmsignals, um den Benutzer aufzuwecken;
wobei die Vorrichtung **dadurch gekennzeichnet ist, dass** sie ferner Folgendes umfasst:
eine Signalumwandlungseinheit (104) zum Umwandeln des extrahierten Schlafgeräuschs in ein digitales Signal;
eine Schlafgeräuschvergleichseinheit (106) zum Vergleichen des umgewandelten Schlafgeräuschs mit einer im Voraus gespeicherten Schlafgeräuschprobe des Benutzers, um die Ähnlichkeit zu prüfen;
eine Alarmsignalausgabe-Steuereinheit (114) zum Steuern der Ausgabe der Alarmsignalausgabeeinheit (120) je nach dem Vergleichsergebnis der Schlafgeräuschvergleichseinheit (106); und
eine Alarmsignalbestimmungseinheit (118) zum Bestimmen einer Art von Alarmsignalausgabe von der Alarmsignalausgabeeinheit (120) auf der Basis von peripheren Umgebungsinformationen als Reaktion auf ein Steuersignal von der Alarmsignalausgabe-Steuereinheit (114).

2. Alarmerzeugungsvorrichtung nach Anspruch 1, wobei die Schlafgeräuschextraktionseinheit (102) die Aufgabe hat, ein menschliches Schnarch- oder Schlafapnoe-Geräusch als das Schlafgeräusch zu extrahieren.

3. Alarmerzeugungsvorrichtung nach Anspruch 1 oder 2, wobei die Schlafgeräuschextraktionseinheit (102) die Aufgabe hat, das extrahierte Schlafgeräusch zu verstärken.

4. Alarmerzeugungsvorrichtung nach einem der vorherigen Ansprüche, wobei die Schlafgeräuschvergleichseinheit (106) die Aufgabe hat, die Schlafgeräuschprobe des Benutzers in einem inneren Speicher (108) oder in einem besonderen Speicher zu speichern und zu verwalten.

5. Alarmerzeugungsvorrichtung nach einem der vorherigen Ansprüche, wobei die Alarmsignalausgabeeinheit (120) die Aufgabe hat, ein Tonsignal, ein Vibrationssignal oder ein Lichtsignal als Alarmsignal auszugeben.

6. Alarmerzeugungsvorrichtung nach einem der vorherigen Ansprüche, wobei die Alarmsignalausgabe-Steuereinheit (114) die Aufgabe hat, Anfang und Ende des Betriebs der Alarmerzeugungsvorrichtung für eine vorbestimmte Zeitperiode in vorbestimmten Intervallen zu steuern.

7. Alarmerzeugungsvorrichtung nach Anspruch 6, wobei die Alarmsignalausgabe-Steuereinheit (114) die Aufgabe hat, Anfang und Ende des Betriebs der Geräuschaufnahmeeinheit (100) und der Bildaufnahmeeinheit (116) als Reaktion auf Anfang und Ende des Betriebs der Alarmerzeugungsvorrichtung zu steuern.

8. Alarmerzeugungsvorrichtung nach einem der vorherigen Ansprüche, wobei die Alarmsignalausgabe-Steuereinheit (114) die Aufgabe hat, die Alarmsignalausgabeeinheit (120) so zu steuern, dass sie kontinuierlich das Alarmsignal ausgibt, während das Schlafgeräusch kontinuierlich aufgenommen wird.

9. Alarmerzeugungsvorrichtung nach einem der vorherigen Ansprüche, die ferner eine Betriebstaste (110) zum Eingeben eines Betriebsanfangs- und Endebefehls durch den Benutzer umfasst.

10. Alarmerzeugungsvorrichtung nach Anspruch 9, wobei die Alarmsignalausgabe-Steuereinheit (114) die Aufgabe hat, Anfang und Ende des Betriebs der Alarmerzeugungsvorrichtung als Reaktion auf die Eingabe des Betriebsanfangs- und Endebefehls über die Betriebstaste (110) zu steuern.

11. Alarmerzeugungsvorrichtung nach Anspruch 9, wobei die Betriebstaste (110) zum Eingeben eines Ausgabestoppbefehls von dem Benutzer dient, der anzeigt, dass die Ausgabe des Alarmsignals von der Alarmsignalausgabeeinheit (120) gestoppt werden soll.

12. Alarmerzeugungsvorrichtung nach Anspruch 1, wobei die Alarmsignalbestimmungseinheit die Aufgabe hat, die Art des Alarmsignals je nach der Wahl des Benutzers zu bestimmen.

13. Alarmerzeugungsvorrichtung nach Anspruch 1, wobei die Alarmsignalbestimmungseinheit die Aufgabe hat, die peripheren Umgebungsinformationen anhand von von einer Bildaufnahmeeinheit zum Aufnehmen von peripheren Bildern aufgenommenen Bildinformationen und anhand von von der Geräuschaufnahmeeinheit aufgenommenen Geräuschinformationen zu erkennen.

14. Alarmerzeugungsvorrichtung nach Anspruch 13, wobei die Alarmsignalbestimmungseinheit (118) die Aufgabe hat, ein Lichtsignal als Alarmsignal zu bestimmen, wenn es dunkel ist.

15. Alarmerzeugungsvorrichtung nach Anspruch 13 oder 14, wobei die Alarmsignalbestimmungseinheit (118) die Aufgabe hat, ein Tonsignal als Alarmsignal zu bestimmen, wenn es still ist.

16. Alarmerzeugungsvorrichtung nach einem der Ansprüche 13 bis 15, wobei die Alarmsignalbestimmungseinheit (118) die Aufgabe hat, ein Vibrationssignal als Alarmsignal zu bestimmen, wenn es laut ist.

17. Mobiltelefon, Notebook-Computer oder Personal Digital Assistant, das/der die Alarmerzeugungsvorrichtung nach einem der vorherigen Ansprüche umfasst.

18. Vorrichtung nach Anspruch 17, wobei die Alarmsignalausgabe-Steuereinheit (114) die Aufgabe hat, den Betrieb der Alarmerzeugungsvorrichtung vorübergehend zu stoppen, wenn das Gerät ein Mobiltelefon oder ein Personal Digital Assistant ist und wenn während des Betriebs der Alarmerzeugungsvorrichtung Funkkommunikation angefordert wird.

19. Vorrichtung nach Anspruch 17 oder 18, wobei die Alarmsignalausgabeeinheit (120) von der Alarmerzeugungsvorrichtung getrennt werden kann und die Aufgabe hat, das Alarmsignal durch Ausführen von Funkkommunikation mit der Alarmerzeugungsvorrichtung auszugeben.

20. Alarmerzeugungsverfahren, das Folgendes beinhaltet:
a. Aufnehmen von peripheren Geräuschen (302);
b. Extrahieren eines menschlichen Schlafgeräuschs aus den aufgenommenen Geräuschen (304);
c. Umwandeln des extrahierten Schlafgeräuschs in ein digitales Signal (306);
d. Vergleichen des umgewandelten Schlafgeräuschs mit einer im Voraus gespeicherten Schlafgeräuschprobe des Benutzers, um die Ähnlichkeit zu prüfen (308); und
e. Ausgeben eines Alarmsignals, um den Benutzer aufzuwecken, wenn das Schlafgeräusch der Schlafgeräuschprobe des Benutzers ähnelt (312),
wobei das Verfahren ferner zwischen d. und e. den folgenden Schritt beinhaltet:
f. Ermitteln einer Art von Alarmsignal auf der Basis der peripheren Umgebungsinformationen (310).

21. Alarmerzeugungsverfahren nach Anspruch 20, wobei in b. ein menschliches Schnarch- oder Schlafapnoe-Geräusch als Schlafgeräusch extrahiert wird.

22. Alarmerzeugungsverfahren nach Anspruch 20 oder 21, wobei in b. das extrahierte Schlafgeräusch verstärkt wird.

23. Alarmerzeugungsverfahren nach einem der Ansprüche 20 bis 22, wobei in e. ein Tonsignal, ein Vibrationssignal oder ein Lichtsignal als Alarmsignal ausgegeben wird.

24. Alarmerzeugungsverfahren nach Anspruch 23, wobei in f. die Art des Alarmsignals gemäß der Wahl des Benutzers ermittelt wird.

25. Alarmerzeugungsverfahren nach Anspruch 23, wobei in f. die peripheren Umgebungsinformationen anhand von Bildinformationen und Geräuschinformationen der peripheren Umgebung erfasst werden.

26. Alarmerzeugungsverfahren nach Anspruch 25, wobei in f. ein Lichtsignal als Alarmsignal bestimmt wird, wenn es dunkel ist.

27. Alarmerzeugungsverfahren nach Anspruch 25 oder 26, wobei in f. ein Tonsignal als Alarmsignal bestimmt wird, wenn es still ist.

28. Alarmerzeugungsverfahren nach einem der Ansprüche 25 bis 27, wobei in f. ein Vibrationssignal als Alarmsignal bestimmt wird, wenn es laut ist.

29. Alarmerzeugungsverfahren nach einem der Ansprüche 20 bis 28, das ferner das Prüfen, ob das Schlafgeräusch kontinuierlich aufgenommen wird, nach Schritt e beinhaltet.

30. Alarmerzeugungsverfahren nach einem der Ansprüche 20 bis 29, wobei das Alarmerzeugungsverfahren für eine vorbestimmte Zeitperiode in vorbestimmten Intervallen ausgeführt wird.

31. Alarmerzeugungsverfahren nach einem der Ansprüche 20 bis 30, wobei der Betrieb des Alarmerzeugungsverfahrens als Reaktion auf einen Betriebsanfangs- und Endebefehls vom Benutzer gestartet und beendet wird.

32. Alarmerzeugungsverfahren nach einem der Ansprüche 20 bis 31, wobei das Alarmerzeugungsverfahren von einem Mobiltelefon, einem Notebook-Computer oder einem Personal Digital Assistant ausgeführt wird.

## Revendications

1. Appareil de génération d'une alarme de réveil comprenant :
une unité de collecte de sons (100) pour collecter des sons périphériques ;
une unité d'extraction de son de sommeil (102) pour extraire un son de sommeil humain des sons collectés ; et
une unité de sortie de signal d'alarme (120) pour produire en sortie un signal d'alarme de réveil pour réveiller l'utilisateur ;
l'appareil étant **caractérisé en ce qu'**il comprend en outré :
une unité de conversion de signal (104) pour convertir le son de sommeil extrait en un signal numérique ;
une unité de comparaison de sons de sommeil (106) pour comparer le son de sommeil converti à un échantillon de son de sommeil d'un utilisateur mémorisé à l'avance pour vérifier la similarité ;
une unité de commande de sortie de signal d'alarme (114) pour commander la sortie de l'unité de sortie de signal d'alarme (120) en fonction du résultat de comparaison de l'unité de comparaison de sons de sommeil (106) ; et
une unité de détermination de signal d'alarme (118) agencée pour déterminer un type de signal d'alarme de réveil produit en sortie par l'unité de sortie de signal d'alarme (120) en fonction d'informations d'environnement périphérique en réponse à un signal de commande provenant de l'unité de commande de sortie de signal d'alarme (114).

2. Appareil de génération d'alarme de réveil selon la revendication 1, dans lequel l'unité d'extraction de son de sommeil (102) est agencée pour extraire un son humain de ronflement ou d'apnée du sommeil comme son de sommeil.

3. Appareil de génération d'alarme de réveil selon la revendication 1 ou 2, dans lequel l'unité d'extraction de son de sommeil (102) est agencée pour amplifier le son de sommeil extrait.

4. Appareil de génération d'alarme de réveil selon l'une quelconque des revendications précédentes, dans lequel l'unité de comparaison de sons de sommeil (106) est agencée pour mémoriser et gérer l'échantillon de son de sommeil de l'utilisateur dans une mémoire interne (108) ou dans une mémoire particulière.

5. Appareil de génération d'alarme de réveil selon l'une quelconque des revendications précédentes, dans lequel l'unité de sortie de signal d'alarme (120) est agencée pour produire en sortie un signal sonore, un signal de vibration ou un signal optique comme signal d'alarme de réveil.

6. Appareil de génération d'alarme de réveil selon l'une quelconque des revendications précédentes, dans lequel l'unité de commande de sortie de signal d'alarme (114) est agencée pour commander le début du fonctionnement et la fin du fonctionnement de l'appareil de génération d'alarme de réveil pendant une période de temps prédéterminée à des intervalles prédéterminés.

7. Appareil de génération d'alarme de réveil selon la revendication 6, dans lequel l'unité de commande de sortie de signal d'alarme (114) est agencée pour commander le début de l'opération et la fin de l'opération de l'unité de collecte de sons (100) et de l'unité de collecte d'images (116), en réponse au début du fonctionnement et à la fin du fonctionnement de l'appareil de génération d'alarme de réveil.

8. Appareil de génération d'alarme de réveil selon l'une quelconque des revendications précédentes, dans lequel l'unité de commande de sortie de signal d'alarme (114) est agencée pour commander à l'unité de sortie de signal d'alarme (120) de produire continûment en sortie le signal d'alarme de réveil tout au long de la collecte continue du son de sommeil.

9. Appareil de génération d'alarme de réveil selon l'une quelconque des revendications précédentes, comprenant en outre un bouton d'actionnement (110) pour l'entrée par l'utilisateur d'une instruction de début de fonctionnement et d'une instruction de fin de fonctionnement.

10. Appareil de génération d'alarme de réveil selon la revendication 9, dans lequel l'unité de commande de sortie de signal d'alarme (114) est agencée pour commander le début du fonctionnement et la fin du fonctionnement de l'appareil de génération d'alarme de réveil en réponse à l'instruction de début de fonctionnement et l'instruction de fin de fonctionnement par le biais du bouton d'actionnement (110).

11. Appareil de génération d'alarme de réveil selon la revendication 9, dans lequel le bouton d'actionnement (110) sert à l'utilisateur à entrer une instruction d'arrêt de sortie indiquant que la sortie du signal d'alarme de réveil depuis l'unité de sortie de signal d'alarme (120) doit être arrêtée.

12. Appareil de génération d'alarme de réveil selon la revendication 1, dans lequel l'unité de détermination de signal d'alarme est agencée pour déterminer le type de signal d'alarme de réveil en fonction de la sélection de l'utilisateur.

13. Appareil de génération d'alarme de réveil selon la revendication 1, dans lequel l'unité de détermination de signal d'alarme est agencée pour détecter les informations d'environnement périphérique à partir d'informations d'images collectées par une unité de collecte d'images servant à collecter des images périphériques et d'informations de sons collectées par l'unité de collecte de sons.

14. Appareil de génération d'alarme de réveil selon la revendication 13, dans lequel l'unité de détermination de signal d'alarme est agencée pour déterminer un signal optique comme signal d'alarme de réveil quand l'environnement est sombre.

15. Appareil de génération d'alarme de réveil selon la revendication 13 ou 14, dans lequel l'unité de détermination de signal d'alarme (118) est agencée pour déterminer un signal sonore comme signal d'alarme de réveil quand l'environnement est silencieux.

16. Appareil de génération d'alarme de réveil selon l'une quelconque des revendications 13 à 15, dans lequel l'unité de détermination de signal d'alarme (118) est agencée pour déterminer un signal de vibration comme signal d'alarme de réveil quand l'environnement est bruyant.

17. Téléphone portable, ordinateur de poche ou assistant numérique personnel comprenant l'appareil de génération d'alarme de réveil selon l'une quelconque des revendications précédentes.

18. Dispositif selon la revendication 17, dans lequel l'unité de commande de sortie de signal d'alarme (114) est agencée pour arrêter temporairement le fonctionnement de l'appareil de génération d'alarme de réveil quand le dispositif est un téléphone portable ou un assistant numérique personnel, et qu'une communication radio est requise durant le fonctionnement de l'appareil de génération d'alarme de réveil.

19. Dispositif selon la revendication 17 ou 18, dans lequel l'unité de sortie de signal d'alarme (120) peut être séparée de l'appareil de génération de signal d'alarme, et est agencée pour produire en sortie le signal d'alarme de réveil en effectuant une communication radio avec l'appareil de génération d'alarme de réveil.

20. Procédé de génération d'alarme de réveil comprenant :
a. la collecte de sons périphériques (302) ;
b. l'extraction d'un son de sommeil humain des sons collectés (304) ;
c. la conversion du son de sommeil extrait en un signal numérique (306) ;
d. la comparaison du son de sommeil converti à un échantillon de son de sommeil d'un utilisateur à l'avance, pour vérifier la similarité (308) ; et
e. la production en sortie d'un signal d'alarme de réveil pour réveiller l'utilisateur quand le son de sommeil est similaire à l'échantillon de son de sommeil de l'utilisateur (312),
le procédé comprenant en outre, entre d. et e.:
f. la détermination d'un type de signal d'alarme de réveil en fonction des informations d'environnement périphérique (310).

21. Procédé de génération d'alarme de réveil selon la revendication 20, dans lequel, en b., un son humain de ronflement ou d'apnée du sommeil est extrait comme son de sommeil.

22. Procédé de génération d'alarme de réveil selon la revendication 20 ou 21, dans lequel, en b., le son de sommeil extrait est amplifié.

23. Procédé de génération d'alarme de réveil selon l'une quelconque des revendications 20 à 22, dans lequel, en e., un signal sonore, un signal de vibration ou un signal optique est produit en sortie comme signal d'alarme de réveil.

24. Procédé de génération d'alarme de réveil selon la revendication 23, dans lequel, en f., le type de signal d'alarme de réveil est déterminé en fonction de la sélection de l'utilisateur.

25. Procédé de génération d'alarme de réveil selon la revendication 23, dans lequel, en f., les informations d'environnement périphérique sont détectées à partir d'informations d'image et d'informations de son de l'environnement périphérique.

26. Procédé de génération d'alarme de réveil selon la revendication 25, dans lequel, en f., un signal optique est déterminé comme signal d'alarme de réveil quand l'environnement est sombre.

27. Procédé de génération d'alarme de réveil selon la revendication 25 ou 26, dans lequel, en f., un signal sonore est déterminé comme signal d'alarme de réveil quand l'environnement est silencieux.

28. Procédé de génération d'alarme de réveil selon l'une quelconque des revendications 25 à 27, dans lequel, en f., un signal de vibration est déterminé comme signal d'alarme de réveil quand l'environnement est bruyant.

29. Procédé de génération d'alarme de réveil selon l'une quelconque des revendications 20 à 28, comprenant en outré :
la vérification si le son de sommeil est collecté ou non continûment, devant être exécutée après e.

30. Procédé de génération d'alarme de réveil selon l'une quelconque des revendications 20 à 29, le procédé de génération d'alarme de réveil étant exécuté pendant une période de temps prédéterminée à des intervalles prédéterminés.

31. Procédé de génération d'alarme de réveil selon l'une quelconque des revendications 20 à 30, dans lequel l'exécution du procédé de génération d'alarme de réveil est lancé et arrêté en réponse à une instruction de début de fonctionnement et une instruction de fin de fonctionnement provenant de l'utilisateur.

32. Procédé de génération d'alarme de réveil selon l'une quelconque des revendications 20 à 31, le procédé de génération d'alarme de réveil étant exécuté par un téléphone portable, un ordinateur de poche ou un assistant numérique personnel.
